# FASCICULE DE BREVET EUROPEEN

(11) **EP 1 348 419 B1**
(45) Date de publication et mention de la délivrance du brevet: **02.01.2008**
(21) Numéro de dépôt: 03290762.8
(22) Date de dépôt: 26.03.2003
(51) Int. Cl.: A61Q 5/06, A61K 8/97

(54) **Utilisation d'un extrait de grenadier pour le maintien de la coloration capillaire**
Verwendung eines Granatapfelbaumextrakts zur Erhaltung der Haarfarbe
Use of a pomegranate tree extract for hair colour retention

(30) Priorité: 27.03.2002 FR 0203831
(43) Date de publication de la demande: 01.10.2003
(73) Titulaire: Pierre Fabre Dermo-Cosmétique, 92100 Boulogne-Billancourt (FR)
(72) Inventeur: Fabre, Bernard, 31450 Belberaud (FR); Ermosilla, Valérie, 31130 Flourens (FR)
(74) Mandataire: Ahner, Francis

(56) Documents cités:
- WO-A-83/00433
- DATABASE CAPLUS [en ligne] CHEMICAL ABSTRACTS SERVICE, COLUMBUS, OHIO, US; retrieved from STN Database accession no. 2001:900524 XP002226227 & JP 2001 342460 A (OGAWA & CO) 14 décembre 2001 (2001-12-14)
- ANONYMOUS: "Colours-hair" INTERNET ARTICLE, [en ligne] XP002226225 Extrait de l'Internet: <URL:http://www.aromantic.co.uk/eshop/prod ucts.asp?category=50> [extrait le 2003-01-03]
- "l'ami des ingrédients naturels-L'encyclopédie" 28 février 1997 (1997-02-28) , ALBAN MULLER XP002226226 * grenadier, paragraphe Utilisations *
- DATABASE WPI Week 199912 Derwent Publications Ltd., London, GB; AN 1999-136879 XP002226229 & JP 11 005975 A (SASEI SEIYAKU KK)
- DATABASE CAPLUS [en ligne] CHEMICAL ABSTRACTS SERVICE, COLUMBUS, OHIO, US; retrieved from STN Database accession no. 2001:717299 XP002226228 & JP 2001 270812 A (PICASO COSMETIC LABORATORY) 2 octobre 2001 (2001-10-02)

## Description

La présente invention concerne des compositions capables de prolonger l'intensité de la couleur des cheveux colorés. Elle concerne en particulier l'utilisation d'un extrait de Grenadier dans ce but.

Le Grenadier porte le nom latin scientifique de *Punica granatum*. C'est un arbuste de 2 à 5 m de haut qui appartient à la famille botanique des Punicaceae. Son origine est probablement la Perse. Le tronc, recouvert d'une mince écorce grisâtre, se ramifie irrégulièrement en branches plus ou moins épineuses portant des feuilles opposées à court pétiole, ovales, entières, vert foncé, luisantes, sans stipule. Les fleurs rouges, pourpres ou grenats sont solitaires à l'aisselle des feuilles ou réunies par groupe de 2 ou 3. Le calice est formé de 4 à 8 sépales, la corolle comprend 4 à 8 pétales, les étamines sont nombreuses et le gynécée est formé de 8 à 9 carpelles soudés au tube du calice. Le fruit globuleux, brun rouge est une baie renfermant de nombreuses graines dont le tégument externe rouge à pulpe acidulée et sucrée constitue la partie comestible de la Grenade (Garnier G., Bezanger-Beauquesne L., Debraux G., Ressources Médicinales de la Flore Française, Vigot Frères Editeurs, 1961, p. 838-842).

Les parties utilisées du Grenadier sont l'écorce des racines et celle du fruit, les fleurs et le suc du fruit. On trouve ces différentes parties décrites dans les anciennes pharmacopées espagnole, américaine et française.

L'écorce des racines de Grenadier n'est plus utilisée aujourd'hui. Son principe actif alcaloïdique, la pelletiérine a des propriétés anthelminthiques particulièrement efficaces contre le tænia. Mais sa toxicité sur l'homme (nausées, vertiges, troubles visuels) a rendu les écorces des racines ainsi que la pelletiérine inusitées.

Le fruit du Grenadier, la Grenade, a bien sûr une utilisation alimentaire. Elle se consomme alors de préférence mûre et bien fraîche, mais elle peut se conserver, comme la pomme et le raisin, fraîche pendant plusieurs mois dans un lieu sec et frais à l'obscurité. C'est à partir de son jus que l'on fabrique un sirop de grenadine rafraîchissant mais qui a été également utilisé comme diurétique et adoucissant contre les inflammations oculaires.

L'écorce du fruit, comme la plupart des parties de la plante, est riche en tanins, environ 25 % d'ellagi-tanin. Ces molécules justifient les utilisations de ces écorces. Ainsi, elles sont utilisées en poudre et en décoction dans le traitement des estomacs atones, les maladies gastro-intestinales et les diarrhées. Dans la leucorrhée et les maladies vaginales, on applique localement des tampons vaginaux imbibés de décoctions concentrées des écorces. En usage externe, les propriétés astringentes et antimicrobiennes des tanins justifient l'utilisation des écorces comme cicatrisant et hémostatique, contre les gingivites et pour l'hygiène buccale en gargarisme.

Certaines applications capillaires sont également décrites dans la chute des cheveux ou pour noircir les cheveux. (Bellakhdar J., La Pharmacopée Marocaine Traditionnelle, Ibis Press 1997, France, p. 450-451).

Enfin, des tanins extraits des écorces sont utilisés en tannerie et pour teindre encore aujourd'hui certains tapis (Cardon D., Du Chatenet G., Guide des Teintures Naturelles, Delachaux et Nietstlé 1990, France, p. 279-280).

Si les fruits du Grenadier et, en particulier, ses écorces ont été utilisés pour colorer en noir les cheveux, aucun travail ne relève leurs propriétés cosmétiques sur les cheveux déjà colorés, en particulier pour prolonger l'intensité de la couleur.
Or, le maintien de la coloration des cheveux fait partie des problèmes rencontrés par les personnes dont les cheveux sont colorés. En effet, au bout de plusieurs lavages, la couleur des cheveux colorés tend à s'affadir et à s'estomper, ce qui nécessite donc une coloration plus fréquente. Or les colorants utilisés malgré les progrès des dernières années, ont toujours des effets secondaires indésirables sur les cheveux et le crâne, ayant tendance à abîmer l'un et irriter l'autre.
De plus, les colorations successives modifient l'état de surface de la fibre capillaire; les écailles qui ont été soulevées lors de la coloration peuvent moins bien se recouvrir les unes les autres, et laissent la fibre capillaire plus sensible aux agressions extérieures.
En conséquence, il est toujours nécessaire de trouver de nouveaux actifs permettant de maintenir la coloration capillaire et ainsi d'éviter l'usage trop fréquent de colorants sur les cheveux.

La présente invention concerne donc l'utilisation d'un extrait de fruit de Grenadier choisi parmi l'extrait de fruit sec ou l'extrait d'écorce de fruit pour le maintien des colorations capillaires et/ou pour prolonger l'intensité de la couleur des cheveux colorés

De façon avantageuse, l'extrait est obtenu par extraction des fruits secs ou écorces de fruit broyées à l'aide d'un solvant choisi parmi l'acétone, un alcool en C₁ à C₄ ou leur mélange avec l'eau.
Avantageusement, le procédé d'extraction est le suivant :
les fruits secs ou les écorces de fruits sèches de Grenadier sont broyés puis extraits dans un solvant qui peut être un alcool de C₁ à C₄, de l'acétone, seuls ou en mélange en toute proportion avec de l'eau. Le rapport plante/solvant peut varier de 1/3 à 1/25. L'extraction est réalisée sous agitation ou de façon statique à une température variant de la température ambiante à la température de reflux pendant une durée allant de 1 à 24 h. Une fois l'extraction réalisée, les solutions sont récupérées par filtration ou par centrifugation. Une deuxième extraction peut être réalisée sur les marcs en respectant les mêmes conditions que la première. Les deux solutions sont alors regroupées. Ces solutions peuvent être concentrées plus ou moins et même être séchées afin d'obtenir une poudre. Elles peuvent conserver leur solvant d'extraction ou être reprises dans un solvant cosmétiquement plus compatible comme le propylène glycol ou le butylène glycol. Les tanins sont dosés à l'aide du réactif de Folin Ciocalteu selon la monographie européenne 4^{ème} édition (1999) : « Détermination des tanins dans les drogues végétales ».

De façon avantageuse, l'extrait de Grenadier comprend 5 à 80 % en poidsⁱ de tanins par rapport au résidu sec de l'extrait, avantageusement 50 à 70% en poids de tanins par rapport au résidu sec de l'extrait.

Dans un mode de réalisation avantageux de l'invention, l'extrait de Grenadier se trouve sous une forme à usage topique, avantageusement sous la forme d'un shampooing, d'un après-shampooing, d'une crème, d'un fluide, d'un spray ou d'un masque.
Avantageusement, l'extrait de Grenadier est utilisé en association avec d'autres composants bien connus de l'homme du métier, tels que des agents tensioactifs, des émulsifiants, des épaississants, des conservateurs, des filtres UV, des parfums, des agents antipelliculaires ou pour la prévention de la chute des cheveux etc.. De façon encore plus avantageuse, l'extrait de Grenadier est présent à une concentration comprise entre 0,1 et 5% en poids, avantageusement entre 0,3 et 3% en poids dans la forme d'administration topique.
Les exemples suivants sont donnés à titre indicatif non limitatifs

### Exemple 1 : préparation d'un extrait de Grenadier

Une tonne d'écorce de fruits de Grenadier sèche et broyée est extraite avec 20 tonnes d'alcool à 96 % à reflux, sous agitation pendant 1 h. La solution est récupérée par filtration. Les tanins sont dosés à une concentration de 0,7 g pour 100 ml, soit 70 g pour 100 g de résidu sec.

### Exemple 2 : préparation d'un extrait de Grenadier

500 kg de fruits secs de Grenadier sont broyés, puis extraits par 3 500 kg d'un mélange eau/acétone en proportion 20/80. L'extraction est menée sous agitation pendant 12 h à 60°C. La solution est recueillie par centrifugation. Cette solution est concentrée, puis évaporée à sec sous vide à 60°C. On obtient ainsi 150 kg d'une poudre après broyage titrant à 50 % en poids en tanin par rapport au résidu sec de l'extrait.

### Exemple 3 : préparation d'un extrait de Grenadier

100 kg d'écorce de fruits de Grenadier broyée sont extraits par 500 kg de méthanol à reflux pendant 2 h. La solution est récupérée par filtration, le marc est re-extrait dans les mêmes conditions avec 250 kg de méthanol, puis la solution est également récupérée par filtration. Les deux solutions sont jointes, puis évaporées sous vide. En cours d'évaporation, on ajoute une tonne de butylène glycol. Le méthanol est évaporé totalement, la solution de butylène glycol est recueillie, filtrée. On obtient ainsi un extrait d'écorce de Grenadier solubilisé dans le butylène glycol. Le dosage des tanins révèle une teneur de 1 %, soit 1 g pour 100 ml d'extrait ce qui correspond à 50 % en poids de tanin par rapport au résidu sec.

### Exemple 4 : évaluation de l'utilisation d'un extrait de Grenadier sur la tenue de la coloration

Un extrait de Grenadier titré en tanin préparé selon l'exemple 1 a été évalué *ex vivo* sur mèches de cheveux par des mesures colorimétriques sur la tenue d'une coloration après des lavages successifs. Pour ce faire, cinq mèches de cheveux châtain ont d'abord été évaluées par la mesure de leur couleur avant coloration. Cette dernière est ensuite réalisée à l'aide d'un gel colorant doux sans oxydant et sans ammoniaque de couleur rouge. Après la pose du colorant, les mèches sont rincées, séchées, et les couleurs sont mesurées. L'extrait de Grenadier est appliqué par trempage sur cinq mèches sur dix, les mèches sont ensuite séchées. On réalise cinq lavages successifs sur toutes les mèches traitées ou non par l'extrait avec un shampoing doux suivi d'un rinçage et d'un séchage. Après chaque série de lavage, des mesures colorimétriques sont réalisées à l'aide d'un chromamètre par des techniques classiques connues de l'Homme du métier. Cette méthode colorimétrique permet de mesurer et de contrôler les couleurs avec exactitude par des paramètres quantitatifs et donc de détecter des différences minimes de couleur entre les échantillons. Le gel colorant appliqué sur les mèches était de couleur rouge, cette dernière correspond au paramètre a* du chromamètre, l'évaluation de la tenue de la coloration est effectuée suivant uniquement ce paramètre.

Les résultats sont exprimés en pourcentage de variation a* par rapport à la couleur de la mèche avant le premier lavage.

| | 1^{er} lavage | 2^{ème} lavage | 3^{ème} lavage | 4^{ème} lavage | 5^{ème} lavage |
|---|---|---|---|---|---|
| Mèches non traitées | - 6 % | - 7 % | - 6 % | - 7 % | - 10 % |
| Mèches traitées | - 4 % | - 2 % | - 3 % | - 3 % | - 5 % |

Dès le premier lavage, les mèches non traitées perdent leur caractère rouge de façon plus importante que les mèches traitées. Au cinquième lavage, l'intensité rouge des mèches non traitées a diminué d'environ 10 %, alors que celle des mèches traitées n'a diminué que de 5 %, c'est-à-dire que la perte de coloration au bout de cinq lavages est deux fois moins importante après traitement des mèches par l'actif.
Ainsi, cette étude permet de montrer que l'application de l'extrait de Grenadier suite à une coloration capillaire tend à optimiser son maintien au cours de lavages successifs.

### Exemple 5 : Shampooing pour cheveux fins et colorés

| (% en poids) | | | |
|---|---|---|---|
| Extrait de Grenadier préparé selon l'exemple 1 | | 1 | % |
| Hydroxypropyltrimmonium guar | | 0,5 | % |
| Sodium laureth sulfate | | 7 | % |
| Cocamidopropyl bétaïne | | 3 | % |
| Protéine de thé cocohydrolysée | | 3 | % |
| Cocamide DEA | | 2 | % |
| Base nacrante | | 3 | % |
| Conservateur, parfum | | QS | % |
| Eau déminéralisée | QSP | 100 | % |
| Acide lactique | QSP | pH 6,5 | |

### Exemple 6 : Shampooing pour cheveux très secs et colorés :

| (% en poids) | | | |
|---|---|---|---|
| Extrait de grenadier préparé selon l'exemple 1 | | 1,5 | % |
| Huile de vison | | 0,3 | % |
| PEG 40 huile de castor hydrogénée | | 0,6 | % |
| Sodium laureth 3 sulfate | | 11 | % |
| Ethylène glycol stéarate | | 1,5 | % |
| Propylène glycol | | 1 | % |
| Hydrotriticum WQ (CRODA) | | 1 | % |
| Cocamide DEA | | 0,75 | % |
| Elfacos GT 282 (AKZO) | | 2 | % |
| Panthénol | | 0,3 | % |
| Polymère polyglycol polyamine | | 0,5 | % |
| Conservateurs, parfum | | QS | |
| Eau déminéralisée | QSP | 100 | |

### Exemple 7 : Shampooing nacre pour cheveux colorés :

| (% en poids) | | | |
|---|---|---|---|
| Extrait de grenadier préparé selon l'exemple 1 | | 2 | % |
| Panthénol | | 0,50 | % |
| Sodium Laureth sulfate | | 7 | % |
| Cocoamphodiacétate (sol.30 %) | | 4 | % |
| Polysorbate 20 | | 4 | % |
| PEG 7 Glycéryl cocoate | | 2 | % |
| PEG 150 distéarate | | 3 | % |
| Base nacrante (sol.20 %) | | QS | % |
| Hydroxypropyltrimmonium guar | | 0,5 | % |
| Acide citrique | QSP | pH 6 | |
| Parfum | | QS | |
| Conservateurs | | QS | |
| Eau déminéralisée | QSP | 100 | |

### Exemple 8 : Masque crème :

| (% en poids) | | | |
|---|---|---|---|
| Extrait de grenadier préparé selon l'exemple 1 | | 3 | % |
| Huile d'Aveline | | 0,75 | % |
| Chlorure de stéapyrium | | 2 | % |
| Paraffine liquide | | 0,75 | % |
| Cétéareth 33 | | 1,3 | % |
| Propylène glycol | | 2 | % |
| Alcool cétéaryl | | 6,7 | % |
| Beurre de Karité | | 0,2 | % |
| Squalane végétal | | 0,1 | % |
| Dioxyde de titane | | 0,1 | % |
| Conservateurs, parfum | | QS | |
| Eau déminéralisée | QSP | 100 | |

### Exemple 9 : Fluide pour pointes sèches :

| (% en poids) | | | |
|---|---|---|---|
| Huile de sésame | | 0,3 | % |
| Extrait de grenadier préparé selon l'exemple 1 | | 0,5 | % |
| Huile de ricin hydrogénée éthoxylée (40 EO) | | 1,5 | % |
| Ethoxydiglycol | | 3 | % |
| Hydroxypropyl méthyl cellulose | | 0,2 | % |
| Acrylate C10-30 alkyl acrylate crosspolymer | | 0,2 | % |
| Cyclométhicone/diméthiconol | | 8 | % |
| Glycérine | | 0,3 | % |
| Hydrolysat de protéine de blé | | 0,3 | % |
| PEG-10 Soya stérol | | 0,05 | % |
| Conservateurs, parfum | | QS | |
| Eau déminéralisée | QSP | 100 | |

### Exemple 10 : Spray démêlant :

| (% en poids) | | | |
|---|---|---|---|
| Extrait de grenadier préparé selon l'exemple 1 | | 1,5 | % |
| Vinaigre d'alcool blanc | QSP | pH 4 | % |
| Chlorure de cétrimonium | | 0,40 | % |
| Diméthicone copolyol | | 0,50 | % |
| Phényltriméthicone | | 0,075 | % |
| Amodiméthicone | | 0,15 | % |
| Parfum | | QS | |
| Eau déminéralisée | QSP | 100 | |

### Exemple 11 : Masque capillaire protecteur solaire :

| (% en poids) | | | |
|---|---|---|---|
| Extrait de grenadier préparé selon l'exemple 1 | | 1,5 | % |
| Steapyrium chloride | | 2 | % |
| Octylméthoxycinnamate | | 1 | % |
| Ceteareth 33 | | 1,3 | % |
| Propylène glycol | | 2 | % |
| alcool de cétéaryl | | 6,7 | % |
| Beurre de karité | | 0,2 | % |
| Squalane végétal | | 0,1 | % |
| Dioxyde de titane | | 0,1 | % |
| Parfum | | QS | % |
| Eau déminéralisée | QSP | 100 | |

## Revendications

1. Utilisation d'un extrait de fruit de Grenadier choisi parmi l'extrait de fruit sec ou l'extrait d'écorce de fruit pour le maintien des colorations capillaires et/ou pour prolonger l'intensité de la couleur des cheveux colorés

2. Utilisation selon la revendication 1, **caractérisée en ce que** l'extrait est obtenu par extraction des fruits secs ou écorces broyés à l'aide d'un solvant choisi parmi l'acétone, un alcool en C₁ à C₄ ou leur mélange avec l'eau.

3. Utilisation selon l'une quelconque des revendications précédentes, **caractérisée en ce que** l'extrait de fruit de Grenadier comprend 5 à 80 % en poids de tanins par rapport au résidu sec de l'extrait, avantageusement 50 à 70% en poids de tanins par rapport au résidu sec de l'extrait.

4. Utilisation selon l'une quelconque des revendications précédentes, **caractérisée en ce que** l'extrait de fruit de Grenadier se trouve sous une forme d'administration topique, avantageusement sous la forme d'un shampooing, d'un après shampooing, d'une crème, d'un fluide, d'un spray ou d'un masque.

5. Utilisation selon la revendication 4, **caractérisée en ce que** l'extrait de fruit de Grenadier est présent à une concentration comprise entre 0,1 et 5% en poids, avantageusement entre 0,3 et 3% en poids dans la forme d'administration topique.

## Claims

1. Use of a pomegranate fruit extract chosen from the dry fruit extract or the fruit peel extract, for hair colour retention and/or for sustaining the intensity of the colour of the coloured hair.

2. Use according to Claim 1, **characterized in that** the extract is obtained by extraction of the ground dried fruits or peel using a solvent chosen from acetone, a C₁ to C₄ alcohol or a mixture thereof with water.

3. Use according to either one of the preceding claims, **characterized in that** the pomegranate fruit extract comprises 5% to 80% by weight of tannins relative to the dry residue of the extract, advantageously 50% to 70% by weight of tannins relative to the dry residue of the extract.

4. Use according to any one of the preceding claims, **characterized in that** the pomegranate fruit extract is in a topical administration form, advantageously in the form of a shampoo, of a conditioner, of a cream, of a fluid, of a spray or of a mask.

5. Use according to Claim 4, **characterized in that** the pomegranate fruit extract is present at a concentration of between 0.1% and 5% by weight, advantageously between 0.3% and 3% by weight, in the topical administration form.

## Patentansprüche

1. Verwendung eines Granatapfelbaumfrucht-Extraktes, der ausgewählt ist aus dem Extrakt von trockener Frucht oder dem Extrakt von Fruchtschale, für die Aufrechterhaltung von Haarfärbungen und/oder um die Intensität der Farbe von gefärbten Haaren zu verlängern.

2. Verwendung nach Anspruch 1, **dadurch gekennzeichnet, dass** der Extrakt durch Extraktion von trockenen Früchten oder gemahlenen Schalen mit Hilfe eines Lösungsmittels erhalten wird, das ausgewählt ist aus Aceton, einem (C₁-C₄)-Alkohol oder deren Mischung mit Wasser.

3. Verwendung nach irgendeinem der vorangehenden Ansprüche, **dadurch gekennzeichnet, dass** der Granatapfelbaumfrucht-Extrakt 5 bis 80 Gew.-% Tannine, bezogen auf den trockenen Rückstand des Extrakts, vorteilhaft 50 bis 70 Gew.-% Tannine, bezogen auf den trockenen Rückstand des Extrakts, umfasst.

4. Verwendung nach irgendeinem der vorangehenden Ansprüche, **dadurch gekennzeichnet, dass** der Granatapfelbaumfrucht-Extrakt in einer topischen Verabreichungsform vorliegt, vorteilhaft in Form eines Shampoos, eines Après-Shampoos, einer Creme, einer Flüssigkeit, eines Sprays oder einer Maske.

5. Verwendung nach Anspruch 4, **dadurch gekennzeichnet, dass** der Granatapfelbaumfrucht-Extrakt in einer Konzentration zwischen 0,1 und 5 Gew.-% einschließlich, vorteilhaft zwischen 0,3 und 3 Gew.-% einschließlich, in der topischen Verabreichungsform vorliegt.
